# NEW EUROPEAN PATENT SPECIFICATION

(11) **EP 2 723 181 B2**
(45) Date of publication and mention of the opposition decision: **28.06.2023**
(45) Mention of the grant of the patent: 18.01.2017
(21) Application number: 12729155.7
(22) Date of filing: 25.06.2012
(51) Int. Cl.: A23C 19/032, A23C 19/076, C12R 1/46

(54) **MANUFACTURE OF COTTAGE CHEESE**
HERSTELLUNG VON HÜTTENKÄSE
FABRICATION DE FROMAGE COTTAGE

(30) Priority: 24.06.2011 DK 201100477 P; 29.07.2011 DK 201100577 P
(43) Date of publication of application: 30.04.2014
(73) Proprietor: Chr. Hansen A/S, 2970 Hørsholm (DK)
(72) Inventor: VAN DEN TEMPEL, Tatjana, DK-2930 Klampenborg (DK); JANZEN, Thomas, DK-2700 Broenshoej (DK); CARLSON, Morten, DK-3200 Helsinge (DK)
(74) Representative: Uexküll & Stolberg
(86) International application number: PCT/EP2012/062274
(87) International publication number: WO 2012/175752

(56) References cited:
- EP-A1- 1 101 407
- WO-A1-01/22828
- WO-A2-99/18807
- WO-A2-2010/023290
- WO-A2-2011/004012
- PERRY D B ET AL: "Manufacture of Low Fat Mozzarella Cheese Using Exopolysaccharide-Producing Starter Cultures", JOURNAL OF DAIRY SCIENCE, AMERICAN DAIRY SCIENCE ASSOCIATION, US, vol. 81, no. 2, 1 February 1998 (1998-02-01), pages 563-566, XP027048101, ISSN: 0022-0302 [retrieved on 1998-02-01]
- HASSAN A N ET AL: "MODIFICATION OF MICROSTRUCTURE AND TEXTURE OF RENNET CURD BY USING A CAPSULE-FORMING NON-ROPY LACTIC CULTURE", JOURNAL OF DAIRY RESEARCH, CAMBRIDGE UNIVERSITY PRESS, CAMBRIDGE, GB, vol. 64, no. 1, 1 January 1997 (1997-01-01), pages 115-121, XP000925734, ISSN: 0022-0299, DOI: 10.1017/S0022029996002002
- LOW D ET AL: "ROLE OF STREPTOCOCCUS THERMOPHILUS MR-1C CAPSULAR EXOPOLYSACCHARIDEIN CHEESE MOISTURE RETENTION", APPLIED AND ENVIRONMENTAL MICROBIOLOGY, AMERICAN SOCIETY FOR MICROBIOLOGY, US, vol. 64, no. 6, 1 June 1998 (1998-06-01), pages 2147-2151, XP000925639, ISSN: 0099-2240
- PERRY D B ET AL: "EFFECT OF EXOPOLYSACCHARIDE-PRODUCING CULTURES ON MOISTURE RETENTION IN LOW FAT MOZZALELLA CHEES", JOURNAL OF DAIRY SCIENCE, AMERICAN DAIRY SCIENCE ASSOCIATION, US, vol. 80, no. 5, 1 May 1997 (1997-05-01), pages 799-805, XP000657588, ISSN: 0022-0302
- WATROUS: "Lactic Cultures In Cottage Cheese", Chr. Hansen, 1997, pages 101-107,
- "Dairy Processing Handbook", Tetra Pak Processing Systems AB, 1995,
- "CHOOZIT MCT Series", DANISCO, 2010,
- DABOUR et al.: "Impact of ropy and capsular exopolysaccharide-producing strains of Lactococcus lactis subsp. cremoris on reduced-fat Cheddar cheese production and whey composition", International Dairy Journal, vol. 15, no. 5, 2015, pages 459-471,
- LOW et al.: "Role of Streptococcus thermophilus MR-1C CapsularExopolysaccharide in Cheese Moisture Retention", Applied and Environmental Microbiology, vol. 64, no. 6, June 1998 (1998-06), pages 2147-2151,
- PETERSEN et al.: "Influence of Capsular and Ropy Exopolysaccharide-ProducingStreptococcus thermophilus on Mozzarella Cheese and Cheese Whey", J. Dairy Sci, vol. 83, no. 9, September 2000 (2000-09), pages 1952-1956,
- BOURDON et al.: "Techniques Bacteriologiques", Biologie Appliquéé, 1973,
- MAMO W.: "Physical and biochemical surf ace properties of Gram-positive bacteria in relation to adhesion to bovine mammary cells and tissues A review of the literature", Rev. sci. tech. Off. Int. Epiz., vol. 8, no. 1, 1989, pages 163-176,
- MENDE S. et al.: "Influence of exopolysaccharides on the structure, texture, stability and sensory properties of yoghurt and related products", International Dairy Journal, vol. 52, January 2016 (2016-01), pages 57-71,
- PACHEKREPAPOL et al.: "Characterization of the chemical structures and physical properties of exopolysaccharides produced by variousStreptococcus thermophilusstrains", J. Dairy Sci., vol. 100, no. 5 , pages 3424-3435,
- BOONAERT et al.: "Surface of Lactic Acid Bacteria: Relationships between Chemical Composition and Physicochemical Properties", Applied and environmental microbiology, vol. 66, no. 6, June 2000 (2000-06), pages 2548-2554,
- Nachtigall et al. Poster at the 12th Symposium on Lactic Acid Bacteria 2017
- Poster Session at the 12th Symposium on Lactic Acid
- Gomez, S. Declaration concerning CPS production of various lactic acid bacteria strains
- RHODIA FOOD BIOLACTA Rhodia cottage cheese production flow chart
- RHODIA FOOD BIOLACTA Rhodia MA series
- RHODIA FOOD BIOLACTA Rhodia RA series
- MCT composition
- Examples of MCT171 culture and MCT172 culture
- Robitaille et al."Detection and Quantification of Capsular Exopolysaccharides from Streptococcus thermophilus Using Lectin Probes?, pages 4156-4162. Journal of Dairy Science, 2006, 89 (11)
- Zisu et al."Effects of pH, Temperature, Supplementation with Whey Protein Concentrate, and Adjunct Cultures on the Production of Exopolysaccharides by Streptococcus thermophilus 1275? Journal of Dairy Science, 2003, 86
- Ceming, J. "Production of exopolysaccharides by lactid acid bacteria and dairy propionbacteria", pages 463-472Lait, 1995, 75
- Fourcassie, P. Declaration concerning CPS production by strain CNCM 1-2311
- Mornet, A.Declaration concerning the date of availabilty of D4
- Banks, J.M. "Cheese problem solved", pages 102-1042007, Chapter 49
- INSTITUTE PASTEUR "Collection nationale de cultures microorganismes" cited 03.04.2019
- Wind/ CHR. HANSEN Request for issue of a sample of deposited biological material signed 12.06.2018
- Response of the EPO to the request for the issue of a sample of bilological material dated 18 08.2018
- Janzen, T. Expert Declaration Thomas Janzen
- EPO Guidelines 2010 part C-ll, 6.3 dated 04.2010
- Decision G 2/9321 December 1994
- Decision T1338/12
- Request filed at the AUSPTO Request for the furnishing of CNCM 1-2311 strain filed at the AUSPTO and certification of release of the CNCM 1-2311 strain provided by the AUSPTO 29 July 2019
- Carlson, M. Expert Declaration Morten Carlson 02. Apr 20
- Chapter 14 of D3
- Experimental data demonstrating CPS production by lactic-acid bacteria
- Confirmation of CHOOZIT MCT 171 composition
- Confirmation of CHOOZIT RA26 composition

## Description

### FIELD OF INVENTION

The present invention relates to the manufacture of cottage cheese by fermenting milk with lactic acid bacteria belonging to a *Lactococcus* species.

### BACKGROUND OF INVENTION

Lactic acid bacteria (LAB) are intensively used in the dairy industry for making various animal milk fermented products, e.g. cheese, especially fresh cheese such as cottage cheese. Cottage cheese accounts for approx. 700,000 tons of the world's 18.2 million tons of cheese consumed in 2008. In North America cottage cheese makes up approx. 12% of all cheese. Normally, cottage cheese cultures comprise homofermentative *Lactococcus* strains such as e.g. *Lactococcus lactis* strains.

As technological background prior art that describes known methods for making cottage cheese as such may herein be mentioned: US3298836 (published 1967); WO91/00690A1 and the article "Gold Spot Dairy boost cottage cheese sales", Dairy and Ice Cream Field, vol. 156, no. 6, 1973, pages 46-47).

Relatively recently (within the last 3-5 years) *Streptococcus thermophilus* (ST) has been added to cottage cheese cultures. Addition of *S*. *thermophilus* may result in a shorter fermentation time (e.g. shortened to around 4-5 hours). However, *S. thermophilus* strains are generally capable of expressing the enzyme urease (EC 3.5.1.5), which is an enzyme that catalyzes the hydrolysis of urea into carbon dioxide (CO₂) and ammonia (NH₃). Milk comprises urea. Accordingly, due to the production of the base NH₃ by *S*. *thermophilus* there may be a temporary decrease in acidification speed during the fermentation of milk. In relation to the problem of NH₃-induced temporary decrease in acidification speed US6962721B1 (Texel, FR) describes that by using *S. thermophilus* strains which e.g. are not producing active urease enzyme (so-called "ur(-) strains") one may get an improved acidification kinetic profile.

Nevertheless, there is a need in the art to provide further improved methods for the manufacture of cottage cheese.

It has now been surprisingly found that during the production of cottage cheese the yield can be significantly improved when using lactic acid bacteria strains belonging to a *Lactococcus* species that produce capsular polysaccharides (CPS). Also, the use of CPS-producing *Lactococcus* strains was found to improve the physical properties of fermented dairy products.

### SUMMARY OF INVENTION

In its broadest aspect, the present invention relates to a novel method for manufacture of cottage cheese, which comprises the following steps:
a) inoculating milk with lactic acid bacteria belonging to a strain of a *Lactococcus* species which is able to produce a capsular polysaccharide (CPS);
b) fermenting the milk with the bacteria; and
c) separating the cheese mass or curd from the whey.

In some embodiments the strain is a strain of *Lactococcus lactis.*

In some embodiments of the method, the strain is urease negative (Ur-).

For example, the strain may be selected from the group consisting of strains that were deposited with the Leibniz Institute DSMZ - German Collection of Microorganisms and Cell Cultures under accession numbers: DSM24650, DSM24648, DSM24649 and DSM21421.

In further embodiments of the method, the milk is inoculated with 10⁴ to 10¹³ cfu/ml (cell forming units per milliliter), or more preferably inoculated with 10⁸ to 10¹² cfu/ml, of the bacteria.

In some embodiments, the method comprises a further step of adding a coagulant (such as rennet, rennin, a protease, and/or chymosin) before, during or after step a).

The fermentation time in step (b) can e.g. be from 3 to 10 hours.

The milk substrate is milk, such as cow's milk.

The step (c) may in some embodiments include one or more of the steps selected from the group consisting of: i) cutting the coagulum at a pH between 4.6 and 4.7; and ii) scalding (heating) of the curd after cutting.

The resulting cheese curd can in some embodiments of the method be salted, pressed and packaged.

The invention also relates to a novel cottage cheese obtained by a method of the invention. The cottage cheese can be distinguished from previously known cottage cheeses, as demonstrated e.g. in the example section below ("Conclusion"). Specifically, the overall mouth feel of the cheese is significantly improved in cheese that has been manufactured by the methods described herein. Further, less residual whey is present in the cheese obtained by the methods of the present invention.

Further, the invention relates to the novel use of CPS-producing bacteria belonging to the species *Lactococcus lactis* in a process for producing cottage cheese, especially the use of a lactic acid bacteria belonging to a strain selected from the group consisting of strains that were deposited with the Leibniz Institute DSMZ - German Collection of Microorganisms and Cell Cultures under accession numbers: DSM24650, DSM24648, DSM24649 and DSM21421.

Finally, the invention relates to a novel lactic acid bacterial strain selected from the group consisting of strains that were deposited with the Leibniz Institute DSMZ - German Collection of Microorganisms and Cell Cultures under accession numbers: DSM24650, DSM24648 and DSM24649.

### DETAILED DISCLOSURE

In a first aspect, the present invention relates to a method for manufacture of cottage cheese, which comprises the following steps:
a) inoculating milk with lactic acid bacteria belonging to a strain of a *Lactococcus* species which is able to produce a CPS (capsular polysaccharide);
b) fermenting the milk with the bacteria; and
c) separating the cheese mass or curd from the whey.

It is contemplated that this method will result in a higher yield of cottage cheese, compared to an identical method, but wherein the strain is not able to produce a CPS. In a particularly preferred embodiment of the invention, the CPS-producing lactic acid bacteria of a *Lactococccus* species used in the above method belong to a strain which is urease negative, or substantially urease negative.

Also disclosed is a method for the manufacture of cheese, which comprises the following steps:
a) inoculating a milk substrate with lactic acid bacteria belonging to a strain which is urease negative, or substantially urease negative;
b) fermenting the milk with the bacteria; and
c) separating the cheese mass or curd from the whey.

It is contemplated that this method will result in a higher yield of cheese, compared to an identical method, but wherein the strain is urease positive.

The above methods will result in a yield that is preferably at least about 1%, about 2%, about 3%, about 4%, about 5%, about 6%, about 7%, about 8%, about 9%, about 10% or more higher than the yield when the same method is carried out with lactic acid bacteria that are not able to produce a CPS. Preferably, the above methods will result in a yield that is between 0.1 and 20%, between 0.5 and 15% or between 1 and 10% higher than the yield when the same method is carried out with lactic acid bacteria that are not able to produce a CPS.

The lactic acid bacteria strain is a member of *Lactococcus* spp. In one embodiment of the method of the invention the strain is a strain of *Lactococcus lactis.*

The *Lactococcus* bacteria used in step (a) of the above method are CPS-producing bacteria. As used herein, the term "CPS-producing" *Lactococcus* bacteria is to be understood as comprising any *Lactococcus* bacterium that is capable of producing a capsular polysaccharide, i.e. a polysaccharide which encloses the bacterial cell to form a capsule. Methods for the detection whether a bacterial strain is a CPS-producing strain are well known in the art. Any suitable method for the detection of saccharides or glycoproteins may be also used to determine whether a lactic acid bacterium is able to produce a CPS. For example, the CPS produced by a lactic acid bacterium may be detected by CPS-specific antibodies. CPS-producing lactic acid bacteria strains can be isolated, e.g. by testing for their hydrophilicity. For example, the distribution of bacterial cells in an organic phase can be compared to their distribution in an aqueous phase. The more cells remain in the aqueous phase, the more hydrophilic the cell surface is, which indicates that a hydrophilic capsular cell envelope is present that is responsible for actively binding water. Without wishing to be bound by theory, it is assumed that it is the water-binding activity of the CPS that is responsible for the increased yield when using CPS-producing lactic acid bacteria strains in a method for making cheese.

In a further embodiment, the strain is selected from the group consisting of strains that were deposited with the Leibniz Institute DSMZ - German Collection of Microorganisms and Cell Cultures under accession numbers DSM24650, DSM24648, DSM24649, and DSM21421. Specifically, the *Lactococcus* bacteria strain may be selected from the group of *Lactococcus lactis* subsp. *lactis* strains that were deposited with the Leibniz Institute DSMZ - German Collection of Microorganisms and Cell Cultures under accession numbers DSM24650 and DSM24648. Further, the *Lactococcus* bacteria strain may be selected from the group of *Lactococcus lactis* subsp. *cremoris* strains that were deposited with the Leibniz Institute DSMZ - German Collection of Microorganisms and Cell Cultures under accession numbers DSM24649 and DSM21421.

In a preferred embodiment, two *Lactococcus* bacteria strains are used in step (a) in the method of the present invention at the same time. For example, two different strains of *Lactococcus lactis* subsp. lactis or two different strains of *Lactococcus lactis* subsp. cremoris may be used at the same time. It is further preferred to use two *Lactococcus lactis* strains, e.g. selected from the group consisting of DSM24650, DSM24648, DSM24649, DSM21421 and DSM21404 at the same time in the methods of the present invention. It is particularly preferred to use *Lactococcus lactis* DSM21404 in combination with another *Lactococcus lactis* strain selected from the group consisting of DSM24650, DSM24648, DSM24649 and DSM21421.

The "milk" of step (a) may be any raw and/or processed milk as described hereinafter. The milk may be inoculated with about 10⁴ to 10¹³ cfu/ml (i.e. cell- or colony-forming units per milliliter), preferably about 10⁶ to 10¹² cfu/ml, or more preferably about 10⁸ to 10¹² cfu/ml, of the bacteria.

The method of the invention may comprise a further step of adding a coagulant, such as rennet, rennin, a protease, and/or chymosin, before, during or after step (a). As described hereinafter, "coagulant" refers to any kind of milk clotting agent. In a preferred embodiment, the coagulant added before, during or after step (a) is bovine chymosin.

In step (b), the milk is fermented with the bacteria as defined hereinafter. A relatively long fermentation period is used in the present methods for the manufacture of cheese to allow a pH drop from around pH 6.6, i.e. the pH of milk, to around 4.65. The fermentation time in step (b) may be in the range from 3 to 10 hours, preferably about 7, about 8, about 9 or about 10 hours. Generally, the fermentation time in step (b) will be selected such that it is sufficiently long to allow a pH drop to about 4.65.

In step (c) the cheese mass or curd is separated from the whey. As defined hereinafter, a "cheese" is a product prepared by contacting milk with a coagulant and draining the resultant curd. Cottage cheese may be seen as a cheese curd product with a mild flavor. It is normally drained but not pressed so some whey may remain and the curd may remain loose. Different types of cottage cheese are made from milk with different fat levels.

The milk can be cow's milk.

In the method of the invention, step (c) may include one or more of the steps selected from the group consisting of:
i) cutting the coagulum at a pH between 4.6 and 4.7; and
ii) scalding (heating) of the curd after cutting.

In one embodiment, step (c) comprises cutting the coagulum at a pH between 4.0 and 5.5, preferably, between 4.3 and 5.0, more preferably between 4.6 and 4.7. The coagulum is preferably cut into small cubes. For this purpose, a cheese wire or frame cutter can be conveniently used. Preferably, the coagulum is cut into cubes having a site of about 10 mm.

In a further embodiment, step (c) comprises scalding or heating of the curd after cutting. In this step, the curd is normally heated to a temperature between about 45-60°C, more preferably 50-55°C, most preferably 56-57°C, and held at that temperature for about 1-2 hours.

The resulting cheese, curd or cheese curd may be salted, pressed, packaged, added further ingredients, such as cream, food colorants, aromas, or otherwise processed. It is known in the art how to select the appropriate additives and/or processing means to arrive at the desired product. It is preferred that the cheese curd, cheese or curd obtained by the present methods is salted, pressed and packaged. Means for pressing and packaging are known in the art.

An aspect of the present disclosure relates to curd obtainable, preferably obtained, by the method of the invention, including the processed curd.

In another aspect, the invention relates to cottage cheese obtained by a method of the present invention.

In yet another aspect, the invention relates to the use of CPS-producing bacteria belonging to the species *Lactococcus lactis* in a process for producing cottage cheese. In a prefered embodiment, the invention relates to the use of a lactic acid bacteria belonging to a strain selected from the group consisting of strains that were deposited with the Leibniz Institute DSMZ - German Collection of Microorganisms and Cell Cultures under accession numbers: DSM24650, DSM24648, DSM24649 and DSM21421 in a process for producing cottage cheese.

In a final aspect, the present invention relates to a lactic acid bacterial strain selected from the group consisting of strains that were deposited with the Leibniz Institute DSMZ - German Collection of Microorganisms and Cell Cultures under accession numbers: DSM24650, DSM24648 and DSM24649.

### DEFINITIONS

In the present context, the term "cheese" refers to a product prepared by contacting milk, optionally acidified milk, such as milk that is acidified e.g. by means of a lactic acid bacterial culture, with a coagulant, and draining the resultant curd. Cheeses and their preparation are described in e.g. "Cheese and Fermented Milk Foods", by Frank V. Kosikowski.

The term "cottage cheese" includes cottage cheeses prepared by any known manufacturing procedure, such as those for instance that are described in the references mentioned herein.

In the present context, the term "coagulant" refers to refers to any kind of milk clotting agent, such as a native enzyme derived from microbial, vegetable or animal tissue sources or a milk clotting enzyme provided as a gene product of recombinant cells expressing a milk clotting enzyme of animal or microbial origin. The term includes bovine chymosin purified from abomasum tissue or made by fermentation (e.g. CHY-MAX (R) or CHY-MAX (R) M). As used herein, the term "lactic acid bacterium" designates a gram-positive, microaerophilic or anaerobic bacterium, which ferments sugars with the production of acids, such as lactic acid, which is the predominantly produced acid, acetic acid and propionic acid. The industrially most useful lactic acid bacteria are found within the order "Lactobacillales" and "Actinomycetales" which include *Lactococcus* spp., *Streptococcus* spp., *Lactobacillus* spp., *Leuconostoc* spp., *Pseudoleuconostoc* spp., *Pediococcus* spp., *Brevibacterium* spp., *Enterococcus* spp. and *Propionibacterium* spp.

In the present context, the term "milk substrate" may be any raw and/or processed milk material that can be subjected to fermentation according to the method of the invention. Thus, useful milk substrates include, but are not limited to, solutions/suspensions of any milk or milk like products comprising protein, such as whole or low fat milk, skim milk, buttermilk, reconstituted milk powder, condensed milk, dried milk, whey, whey permeate, lactose, mother liquid from crystallization of lactose, whey protein concentrate, or cream. Obviously, the milk substrate may originate from any mammal, e.g. being substantially pure mammalian milk, or reconstituted milk powder. Preferably, at least part of the protein in the milk substrate is proteins naturally occurring in milk, such as casein or whey protein. However, part of the protein may be proteins which are not naturally occurring in milk. Prior to fermentation, the milk substrate may be homogenized and pasteurized according to methods known in the art.

The term "milk" is to be understood as the lacteal secretion obtained by milking any mammal, such as cows, sheep, goats, buffaloes or camels. In a preferred embodiment, the milk is cow's milk. The term milk also comprises non-animal milks, such as soy milk. Optionally, the milk is acidified, e.g. by addition of an acid (such as citric, acetic or lactic acid), or mixed, e.g. with water. The milk may be raw or processed, e.g. by filtering, sterilizing, pasteurizing, homogenizing, etc., or it may be reconstituted dried milk. An important example of "bovine milk" according to the present invention is pasteurized cow's milk. It is understood that the milk may be acidified, mixed or processed before, during and/or after the inoculation with bacteria.

"Homogenizing" as used herein means intensive mixing to obtain a soluble suspension or emulsion. If homogenization is performed prior to fermentation, it may be performed so as to break up the milk fat into smaller sizes so that it no longer separates from the milk. This may be accomplished by forcing the milk at high pressure through small orifices.

"Pasteurizing" as used herein means treatment of the milk substrate to reduce or eliminate the presence of live organisms, such as microorganisms. Preferably, pasteurization is attained by maintaining a specified temperature for a specified period of time. The specified temperature is usually attained by heating. The temperature and duration may be selected in order to kill or inactivate certain bacteria, such as harmful bacteria. A rapid cooling step may follow.

"Fermentation" in the methods of the present invention means the conversion of carbohydrates into alcohols or acids through the action of a microorganism. Preferably, fermentation in the methods of the invention comprises conversion of lactose to lactic acid.

Fermentation processes to be used in production of fermented milk products are well known and the person of skill in the art will know how to select suitable process conditions, such as temperature, oxygen, amount and characteristics of microorganism(s) and process time. Obviously, fermentation conditions are selected so as to support the achievement of the present invention, i.e. to obtain a fermented milk product.

Lactic acid bacteria, including bacteria of the species *Lactococcus lactis* and *Streptococcus thermophilus,* are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product, such as a fermented milk product. Such cultures are in general referred to as "starter cultures" or "starters".

In the present context, the term "packaged" relates to the final packaging of the product to obtain a product that can be supplied to a consumer. A suitable package may thus be a bag, e.g. a sealed plastic bag, a bottle, a container or similar, e.g. containing from 10 g to 5000 g, but it is presently preferred that a package contains from 50 g to 1000 g.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

### EXAMPLES

### Example 1: Standard Cottage Cheese Manufacture

The skilled person knows how to produce cottage cheese and how it differentiates from other cheese (e.g. a soft cheese). A standard manufacture of cottage cheese providing a high cheese yield is described herein below.

Fermentation: Milk was fermented/coagulated 5-6 hours at 34°C for short-set manufacture, whereby the fermentation time depended on the inoculation rate and the type of culture.

Cutting the curd: The pH was approximately 4.65 at the time the curd was cut (a pH>4.7 would have caused matting of the curd). The coagulum was cut by a frame cutter with 10 mm between the strings. First, the frame cutter was run 2 times vertically from side to side and then vertically from end to end followed by a horizontal run from end to end in the cheese vat until cubes of 10 mm were obtained.

The degree and type of agitation of curds after cutting and during cooking influences the end product greatly also in regards to yield:
Healing: During this time, 30 min., the curd was not agitated. The curd structure was fragile. The first agitation did not occur until 50 min. after starting the heating (cooking).
Cooking: The cooking was done step by step, at an approximate rate of 1°C per 5 minutes.

The curd was cooked to expel the whey and to firm the individual curd particles. This was done slowly and with sufficient agitation to prevent the curds from matting, but gentle enough to prevent shattering of the curds.

This gave a uniform firming from surface to center in the curds throughout the vat and prevented matting and the formation of tough skin during cooking.

As mentioned above, the agitator was switched on 50 min after the start of cooking. 5 min later the temperature in the vat was measured (the temperature reached the target temperature of 38°C).

The cooking and stirring process was continued until the temperature was 57°C in the vat approximately 110-120 min. after cooking was started.
The whey was drained until the curd appeared and the jacket was drained.

Washing: The pH in the water for washing was adjusted to 5.0-5.2 with phosphoric acid and 3 ppm hypochlorite (0.18 ml/60 l) were added as well.
Wash 1: the jacket was drained and 60 l water at 12-13°C were added to the vat (in this example = 100 liter vat) and stirred for 15 min. - then the jacket was drained and filled with cold water.
Wash 2: 60l water at 12-13°C were added and stirred for 30 min. and then drained completely (trenching).

The drain was checked after trenching (10-20 min.) with a hand-held refractometer and a Brix of 1.0% ± 0.2 was found. (A Brix of greater than 1.2 could possibly indicate that not enough whey was removed on the initial drain prior to the first 60 liter wash.)

### Cream/dressing for Cottage Cheese

The dressing was prepared the day before the production day.

A creaming ratio, i.e. curd to dressing of 60:40 was used. Other creaming ratios may also be employed.

A cream with 10.5% fat was made, wherein cream with 13-18% fat and skim milk was used. Optionally, 1-2% SMP may be added, and heated up to 50°C and homogenized at 150 bars. Pasteurization at 90°C for 20 min. and subsequent cooling to 10°C may be carried out. Further optionally NaCl (0.8-3.5%) may be added and the dressing may be stored at 3-5°C till the next day.

### Mixing Curd / Dressing

The curd and dressing were weighed and mixed with each other very carefully in a plastic box (40x60 cm).

### Example 2: Novel strains of Lactococcus lactis subsp. Lactics and Lactococcus lactis subsp. Cremoris increase cheese yield in a cottage cheese make.

Two novel strains of *Lactococcus lactis* subsp. *lactis* (DSM24650 and DSM24648) and two novel strains of *Lactococcus lactis* subsp. *cremoris* (DSM24649 and DSM21421) were tested in the standard procedure for making cottage cheese as described in example 1.

In order to compare the **extra** yield obtained by the use of these 4 mesophilic strains - the following setup was conducted:
A 50:50 combination of the *Lactococcus lactis* strain DSM21404 with the *Streptococcus thermophilus* strain DSM24653 was used as a reference blend.

Reference blend (inoculation 0.030% in skim milk):

### 1A) 50% Lactococcus lactis DSM21404 + 50% Streptococcus thermophilus DSM24653

### Test blends (inoculation 0.030% in skim milk) - focus on extra yield from Lactococcus strains:

2) 50% *Lactococcus lactis* DSM24650 + 50% *Lactococcus lactis* DSM21404
3) 50% *Lactococcus lactis* DSM24648 + 50% *Lactococcus lactis* DSM21404
4) 50% *Lactococcus lactis* DSM24649 + 50% *Lactococcus lactis* DSM21404
5) 50% *Lactococcus lactis* DSM21421 + 50% *Lactococcus lactis* DSM21404

This reference blend and the test blends were tested together in a Cottage Cheese manufacture that was completely identical:
After removing the whey and draining of the product, the total cheese weight was calculated. The 'extra yield (%)', i.e. the additional yield in comparison to the reference blend, was calculated in percent using the reference blend as a baseline. A summary of the 'extra yield' in percent for the novel strains of *Lactococcus lactis* can be seen in the Table below:

| Blend | Strain | **Extra** yield (%) |
|---|---|---|
| 1A Reference | DSM21404 | |
| 2 | DSM24649 | >6% |
| 3 | DSM24648 | >1% |
| 4 | DSM24650 | >9% |
| 5 | DSM21421 | >10% |

Based on these investigations it is clearly demonstrated that above *Lactococcus* strains have the ability to increase the cheese yield as tested in a cottage cheese make.

### Example 3: Streptococcus thermophilus increasing cheese yield during the manufacture of cottage cheese (for illustrative purposes only)

Similar investigations were carried out for two strains of *Streptococcus thermophilus* (DSM24655 and DSM24654).

In order to compare the **extra** yield, i.e. the additional yield, obtained by using these 2 thermophilic *Streptococcus thermophilus* strains the following setup was conducted, using the same reference blend that was used in Example 2:

### Reference blend (inoculation 0.030% in skim milk):

1B) 50% *Lactococcus lactis* DSM21404 + 50% *Streptococcus thermophilus* DSM24653

### Test blends (inoculation 0.030% in skim milk) - focus on extra yield from S. thermophilus strains:

6) 50% *Streptococcus thermophilus* DSM24655 + 50% *Streptococcus thermophilus* DSM24653
7) 50% *Streptococcus thermophilus* DSM24654 + 50% *Streptococcus thermophilus* DSM24653

This reference blend and the test blends were tested together in a cottage cheese manufacture that was completely identical.

After removing the whey and draining of the product, the total cheese weight was calculated. The 'extra yield (%)', i.e. the additional yield in comparison to the reference blend, was calculated using the reference blend as a baseline. A summary of the 'extra yield' in percent for the novel strains of Streptococcus thermophilus can be seen in the Table below:

| Blend | Strain | **Extra** yield (%) |
|---|---|---|
| 1B Reference | DSM24653 | |
| 6 | DSM24655 | > 6% |
| 7 | DSM24654 | > 6% |
| 8 | ? | > 2% |

Based on these investigations it is clearly demonstrated that above *S. thermophilus* strains have the ability to increase the cheese yield as tested in the standard cottage cheese manufacture.

### Conclusion:

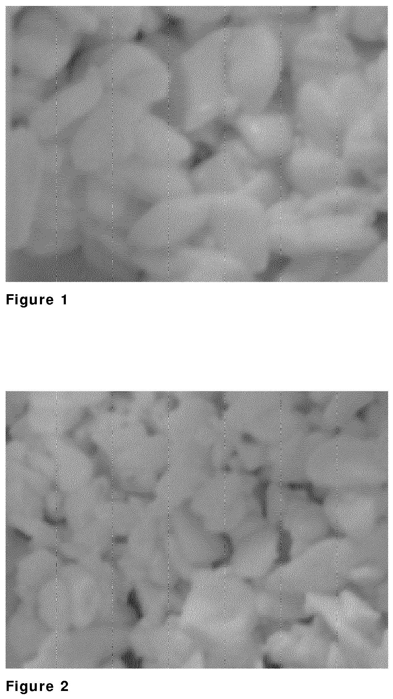

It is clearly demonstrated that some *L. lactis.* subsp *lactis*/subsp. *cremoris* and some *S. thermophilus* strains have a positive impact on cheese yield. In addition a significant quality difference was observed when comparing the cheese curd produced by the reference cultures as compared to the 'extra yield' cultures (examples 2+3):
1) Unexpectedly large cheese curds were observed when using the described 'extra yield strains' of *Streptococcus thermophilus* (ST) or *Lactococcus lactis* subspecies *lactis*/*cremoris.* In addition the curd was as firm as the reference and was very easy to handle.
2) In addition the curd demonstrated to have improved skin, which from a production point of view is very important for the curd handling.
3) As visualised on the figures 1 (cottage cheese manufactured with strain DSM24649) and 2 (cottage cheese manufactured with reference culture DSM21404) the curd had less fines, i.e. small particles. This means that the loss of fine curd particles during draining was minimised thereby increasing the yield.

The improvement of the described strains also had a clear impact on the final product, i.e. the curd plus dressing.
1) The overall mouth feel of the cottage cheese was significantly improved by using the investigated strains as compared to the reference culture.
2) Less residual whey was observed on the cottage cheese made with the novel strains.
3) When stirring in the final product - the reference blends were more fragile (curd broke down into fines) as compared to cottage cheese produced with *Streptococcus thermophilus* or *Lactococcus lactis* subspecies *lactis*/*cremoris* as described herein.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

### DESCRIPTION OF THE FIGURES

The figures depict the impact of the novel strains on curd quality.
Figure 1: Cottage cheese curd with extra yield manufactured with novel strain (DSM24649)
Figure 2: Cottage cheese curd - manufactured with reference culture (DSM21404)

### DEPOSITS and EXPERT SOLUTION

The following strains were deposited at the Leibniz Institute DSMZ - German Collection of Microorganisms and Cell Cultures (formerly known as DSMZ - Deutsche Sammlung von Mikroorganismen und Zellkulturen GmbH), Inhoffenstr. 7B, D-38124 Braunschweig (DSM) on the mentioned dates, and given the mentioned accession numbers:
Novel strains:
   CHCC10812: 2011-03-15, DSM24650
   CHCC6086: 2011-03-15, DSM24648
   CHCC2618: 2011-03-15, DSM24649
   CHCC14308: 2011-03-15, DSM24655
   CHCC12828: 2011-03-15, DSM24654
   CHCC2907: 2008-04-23, DSM21421 (deposit mentioned in WO 2010/023290 A2)
   CHCC12942: July 12, 2011, DSM 25012
Reference strains:
   CHCC12406: 2011-03-15, DSM24653
   CHCC1915: 2008-04-23, DSM21404

The deposits were made according to the Budapest treaty on the international recognition of the deposit of microorganisms for the purposes of patent procedure.

The Applicant requests that a sample of the deposited microorganisms should be made available only to an expert approved by the Applicant.

### REFERENCES

US5116737
US6962721B1 (Texal, FR)
US3298836 (published 1967)
WO91/00690A1
"Gold Spot Dairy boost cottage cheese sales", Dairy and Ice Cream Field, vol. 156, no. 6, 1973, pages 46-47.
R. Scott, (1986), Cheesemaking process, second ed., Elsevier Applied Science Publishers, London and New York.
G. Bylund, (1995), Dairy processing handbook, Tetra Pak Processing Systems, Lund, Sweden F. Kosikowski, (1982), Cheese and fermented milk foods, second ed., Kosikowski & Associates, New York

## Claims

1. A method for manufacture of cottage cheese, which comprises the following steps:
a) inoculating milk with lactic acid bacteria belonging to a strain of a *Lactococcus* species which is able to produce a capsular polysaccharide;
b) fermenting the milk with the bacteria; and
c) separating the cheese mass or curd from the whey.

2. The method of claim 1, wherein the strain is a strain of *Lactococcus lactis.*

3. The method of the preceding claim, wherein the strain is urease negative (Ur-).

4. The method of claim 1, wherein the strain is selected from the group consisting of strains that were deposited with the Leibniz Institute DSMZ - German Collection of Microorganisms and Cell Cultures under accession numbers DSM24650, DSM24648, DSM24649 and DSM21421.

5. The method of any of the preceding claims, wherein the milk is inoculated with 10⁴ to 10¹³ cfu/ml (cell forming units per milliliter), or more preferably inoculated with 10⁸ to 10¹² cfu/ml, of the bacteria.

6. The method of any of the preceding claims, which comprises a further step of adding a coagulant, such as rennet, rennin, a protease, and/or chymosin, before, during or after step a).

7. The method of any of the preceding claims, wherein the fermentation time in step (b) is from 3 to 10 hours.

8. The method of claim 1 or 2, wherein the milk is cow's milk.

9. The method of any of the preceding claims, wherein the step (c) includes one or more of the steps selected from the group consisting of:
i) cutting the coagulum at a pH between 4.6 and 4.7; and
ii) scalding of the curd after cutting.

10. The method of any of the preceding claims, wherein the resulting cheese curd is salted, pressed and packaged.

11. Cottage cheese, obtained by a method of any preceding claim.

12. Use of capsular polysaccharide producing bacteria belonging to the species *Lactococcus lactis* in a process for producing cottage cheese.

13. The use of claim 12, wherein said capsular polysaccharide producing bacteria are lactic acid bacteria belonging to a strain selected from the group consisting of strains that were deposited with the Leibniz Institute DSMZ - German Collection of Microorganisms and Cell Cultures under accession numbers DSM24650, DSM24648, DSM24649 and DSM21421.

14. A lactic acid bacterial strain selected from the group consisting of strains that were deposited with the Leibniz Institute DSMZ - German Collection of Microorganisms and Cell Cultures under accession numbers DSM24650, DSM24648 and DSM24649.

## Patentansprüche

1. Verfahren zur Herstellung von Hüttenkäse, bei dem man:
(a) Milch mit Milchsäurebakterien inokuliert, die zu einem Stamm einer *Lactococcus*-Spezies gehören, der in der Lage ist, ein Kapsel-Polysaccharid zu erzeugen,
(b) die Milch mit den Bakterien fermentiert; und
(c) die Käsemasse oder den Käsebruch von der Molke abtrennt.

2. Verfahren gemäß Anspruch 1, bei dem der Stamm ein Stamm der Spezies *Lactococcus lactis.*

3. Verfahren gemäß einem der vorhergehenden Ansprüche, bei dem der Stamm Urease negativ (Ur-) ist.

4. Verfahren gemäß Anspruch 1, bei dem der Stamm ausgewählt ist aus der Gruppe bestehend aus Stämmen, die beim Leibniz Institut DSMZ - Deutsche Sammlung von Mikroorganismen und Zellkulturen mit den Hinterlegungsnummern DSM24650, DSM24648, DSM24649 und DSM21421 hinterlegt sind.

5. Verfahren gemäß einem der vorhergehenden Ansprüche, bei dem die Milch mit 10⁴ bis 10¹³ KBE/ml (koloniebildenden Einheiten pro Milliliter), oder stärker bevorzugt mit 10⁸ bis 10¹² KBE/ml, der Bakterien inokuliert wird.

6. Verfahren gemäß einem der vorhergehenden Ansprüche, bei dem ferner vor, während oder nach Schritt (a) ein Koagulans, wie z.B. Lab, Rennin, eine Protease und/oder Chymosin zugefügt wird.

7. Verfahren gemäß einem der vorhergehenden Ansprüche, bei dem die Fermentationszeit in Schritt (b) 3 bis 10 Stunden beträgt.

8. Verfahren gemäß Anspruch 1 oder 2, bei dem die Milch Kuhmilch ist.

9. Verfahren gemäß einem der vorhergehenden Ansprüche, bei dem Schritt (c) einen oder mehrere Schritte umfasst, die ausgewählt sind aus der Gruppe bestehend aus:
i) Schneiden des Koagulums bei einem pH zwischen 4,6 und 4,7; und
ii) Erhitzen der Käsebruchs nach dem Schneiden.

10. Verfahren nach einem der vorhergehenden Ansprüche, bei dem der entstandene Käsebruch gesalzen, gepresst und verpackt wird.

11. Hüttenkäse, der mittels des Verfahrens gemäß einem der vorhergehenden Ansprüche erhalten wurde.

12. Verwendung von Bakterien, die ein Kapsel-Polysaccharid erzeugen und der Spezies *Lactococcus lactis* angehören, in einem Verfahren zur Herstellung von Hüttenkäse.

13. Verwendung gemäß Anspruch 12, bei der die Bakterien, die ein Kapsel-Polysaccharid erzeugen, Milchsäurebakterien sind, die zu einem Stamm gehören, der ausgewählt ist aus der Gruppe bestehend aus Stämmen, die beim Leibniz Institut DSMZ - Deutsche Sammlung von Mikroorganismen und Zellkulturen mit den Hinterlegungsnummern DSM24650, DSM24648, DSM24649 und DSM21421 hinterlegt sind.

14. Milchsäurebakterienstamm, der ausgewählt ist aus der Gruppe bestehend aus Stämmen, die beim Leibniz Institut DSMZ - Deutsche Sammlung von Mikroorganismen und Zellkulturen mit den Hinterlegungsnummern DSM24650, DSM24648 und DSM24649 hinterlegt sind.

## Revendications

1. Méthode pour la fabrication de fromage cottage, comprenant les phases suivantes :
a) inoculation à du lait de bactéries d'acide lactique appartenant à une souche d'une espèce *Lactococcus* qui est capable de produire un polysaccharide capsulaire ;
b) fermentation du lait avec les bactéries ; et
c) séparation de la masse de fromage ou caillé du petit-lait.

2. Méthode selon la revendication 1, où la souche est une souche de *Lactococcus lactis.*

3. Méthode selon la revendication précédente, où la souche est uréase négative (Ur-).

4. Méthode selon la revendication 1, où la souche est sélectionnée du groupe composé de souches qui ont été déposées au Leibniz Institute DSMZ - Collection Allemande de Microorganismes et de Cultures Cellulaires sous les numéros d'entrée : DSM24650, DSM24648, DSM24649 et DSM21421.

5. Méthode selon l'une quelconque des revendications précédentes, où 10⁴ à 10¹³ ufc/ml (unités formant des colonies par millilitre), ou plus préférablement 10⁸ à 10¹² ufc/ml des bactéries sont inoculés dans le lait.

6. Méthode selon l'une quelconque des revendications précédentes, comprenant une phase additionnelle d'addition d'un coagulant, comme par exemple présure, rennine, une protéase, et/ou chymosine, avant, pendant ou après la phase a).

7. Méthode selon l'une quelconque des revendications précédentes, où le temps de fermentation dans la phase (b) est 3 à 10 heures.

8. Méthode selon la revendication 1 ou 2, où le lait est du lait de vache.

9. Méthode selon l'une quelconque des revendications précédentes, où la phase (c) inclut une ou plusieurs des phases sélectionnées du groupe composé de :
i) découpage du coagulum à un pH entre 4.6 et 4.7 ; et
ii) ébouillantage du caillé après le découpage.

10. Méthode selon l'une quelconque des revendications précédentes, où le fromage caillé résultant est salé, pressé et mis sous emballage.

11. Fromage cottage, obtenu par une méthode selon l'une quelconque des revendications précédentes.

12. Utilisation de bactéries produisant un polysaccharide capsulaire appartenant à l'espèce *Lactococcus lactis* dans un procédé pour la production de fromage cottage.

13. Utilisation selon la revendication 12, où lesdites bactéries produisant un polysaccharide capsulaire sont des bactéries d'acide lactique appartenant à une souche sélectionnée du groupe composé de souches qui ont été déposées au Leibniz Institute DSMZ - Collection Allemande de Microorganismes et de Cultures Cellulaires sous les numéros d'entrée DSM24650, DSM24648, DSM24649 et DSM21421.

14. Souche bactérienne d'acide lactique sélectionnée du groupe composé de souches qui ont été déposées au Leibniz Institute DSMZ - Collection Allemande de Microorganismes et de Cultures Cellulaires sous les numéros d'entrée DSM24650, DSM24648 et DSM24649.
